(19) Europäisches Patentamt
European Patent Office
Office européen des brevets

(11) Veröffentlichungsnummer: **0 226 101**
**B1**

(12) # EUROPÄISCHE PATENTSCHRIFT

(45) Veröffentlichungstag der Patentschrift: **31.01.90**

(51) Int. Cl.⁴: **C 07 J 31/00, C 07 J 53/00**

(21) Anmeldenummer: **86116565.2**

(22) Anmeldetag: **28.11.86**

(54) **Verfahren zur Herstellung von 7-alpha,Acetylthiosteroiden.**

(30) Priorität: **13.12.85 DE 3544661**

(43) Veröffentlichungstag der Anmeldung: **24.06.87 Patentblatt 87/26**

(45) Bekanntmachung des Hinweises auf die Patenterteilung: **31.01.90 Patentblatt 90/5**

(84) Benannte Vertragsstaaten: **AT BE CH DE ES FR GB GR IT LI LU NL SE**

(56) Entgegenhaltungen:
**EP-A- 0 099 853**
**DE-A- 2 809 838**
**FR-A- 2 370 755**
**US-A- 3 013 012**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Nickisch, Klaus, Dr., Alt Lichtenrade 11, D-1000 Berlin 49 (DE)**
Erfinder: **Laurent, Henry, Dr., Glambecker Weg 21, D-1000 Berlin 28 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 226 101 B1

ACTORUM AG

**Beschreibung**

Die vorliegende Erfindung betrifft ein Verfahren zur Herstellung von 7α-Acetylthiosteroiden.

Das wohl bekannteste 7α-Acetylthiosteroid Spironolacton stellt ein bewährtes Diuretikum dar. Alle bekannten Herstellungsverfahren für Spironolacton verlaufen über 3-Oxo-17α-pregna-4,6-dien-21,17-carbolacton, an das in letzter Stufe Thioessigsäure addiert wird. Dabei treten als Nebenprodukt stets ca. 7% des unwirksamen 7β-Isomeren auf. Aus all diesen Verfahrensprodukten muß das unerwünschte 7β-Isomere erst durch aufwendige Reinigungsmethoden abgetrennt werden, was zwangsläufig zu Ausbeuteverlusten an Spironolacton führt.

Aus DE-OS 2 809 838 ist ein Verfahren bekannt, wonach die Addition von Thioessigsäure an 3-Oxo-17α-pregna-4,6-dien-21,17-carbolacton in Gegenwart einer starken Säure wie z.B. p-Toluolsulfonsäure, bei erhöhter Temperatur durchgeführt wird. Doch auch mit diesem Verfahren läßt sich der Anteil an 7β-Isomeren nicht unter 7,5% drücken. Dieses Verfahren kann außerdem nicht auf die Synthese des Mespirenons angewandt werden, da die verwendeten drastischen sauren Bedingungen an einer Umwandlung des säurelabilen 15β,16β-Methylenspirolactons führen.

Es wurde nun gefunden, daß man die Bildung von 7β-Acetylthioderivaten fast vollkommen unterdrücken kann, wenn man die Thioessigsäureanlagerung in Gegenwart von Lewissäuren durchführt.

Gegenstand der Erfindung ist somit ein Verfahren zur Herstellung von 7α-Acetylthiosteroiden der Formel I

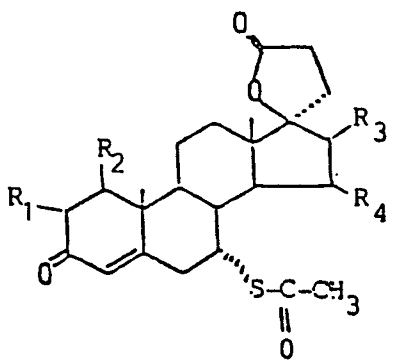

(I),

worin $R_1$, $R_2$, $R_3$ und $R_4$ Wasserstoff oder

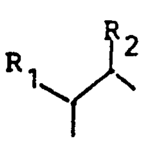 die Gruppe 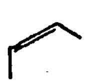 und

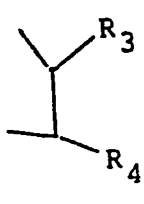 die Gruppe 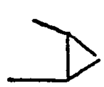 bedeuten,

indem man ein 3-Oxo-17α-pregna-4,6-dien-21,17-carbolacton der Formel II

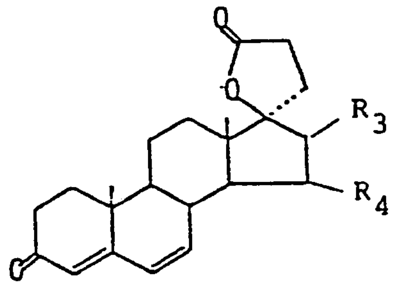

(II),

worin $R_3$ und $R_4$ die bereits genannten Bedeutungen haben, mit Thioessigsäure umsetzt, und das erhaltene Produkt gegebenenfalls dehydriert, das dadurch gekennzeichnet ist, daß die Addition von Thioessigsäure in Gegenwart einer Lewissäure durchgeführt wird.

Als Lewissäuren kommen alle aus der einschlägigen Literatur bekannten Lewissäuren in Betracht. Genannt seinen $SnCl_4$, $TiCl_4$, $ZnCl_2$, $BF_3$-Etherat usw. Vorzugsweise findet $BF_3$-Etherat Verwendung.

Als Lösungsmittel kommen sowohl Alkohole, wie Methanol, Ethanol oder Isopropanol, als auch cyclische Äther, wie Tetrahydrofuran oder Dioxan in Betracht. Die Reaktionstemperaturen bewegen sich zwischen 0° und Siedepunkt des verwendeten Lösungsmittels, vorzugsweise bei 0-75°C.

Unter diesen Bedingungen beträgt der Anteil der 7β-Isomeren in der Spironolactonreihe im Rohprodukt nach HPLC-Analytik weniger als 1%, während in der 15β,16β-Methylenspirolactonreihe nur noch 0,6% des 7β-Isomeren im Rohprodukt nachgewiesen werden können.

Die Endreinigung der gewünschten 7α-Isomeren gelingt problemlos über eine Umkristallisation.

Die Dehydrierung des aus 15β,16β-Methylen-3-oxo-17α-pregna-4,6-dien-21,17-carbolacton erhaltenen 1,2-Dihydro-mespirenons wird nach bekannten Verfahren durchgeführt (z.B. mit DDQ, $SeO_2$).

*Beispiel 1*

Zu einer Suspension von 50 g 3-Oxo-17α-pregna-4,6-dien-21,17-carbolacton in 500 ml i-Propanol gibt man bei Raumtemperatur unter Argon 18 ml $BF_3$-Etherat und 12,5 ml Thioessigsäure. Die Suspension wird bei 60°C 30 Minuten gerührt, in Kochsalz-gesättigtem Wasser gefällt, abgesaugt, mit Wasser gewaschen und im Vakuum getrocknet. Man erhält 58,9 g Rohprodukt, das aus Methanol umkristallisiert wird und 51,5 g Spironolacton ergibt.

$[\alpha_D] = -36{,}5$ UV: $e_{237} = 18750$ Fp.: 195,9°C

*Beispiel 2*

Eine Lösung von 50 g 15β,16β-Methylen-3-oxo-17α-pregna-4,6-dien-21,17-carbolacton (HPLC-Gehalt 81%) in 500 ml i-Propanol wird auf 0°C abgekühlt, mit 18 ml $BF_3$-Etherat und 12,5 ml Thioessigsäure versetzt und eine Stunde bei dieser Temperatur nachgerührt. Gegen Ende der Reaktion begann die Substanz auszufallen. Die Substanz wurde in 5 l mit Kochsalz-gesättigtem Eiswasser gefällt und mit gesättigter Natriumhydrogencarbonatlösung auf

pH = 7 eingestellt. Der ausgefallene Niederschlag wird abgesaugt, mit Wasser gewaschen und im Vakuum getrocknet. Man erhält 47,1 g 1,2-Dihydromespirenon, das nach HPLC-Analyse 0,6% des 7β-Isomeren enthält.

**Patentansprüche**

1. Verfahren zur Herstellung von 7α-Acetylthiosteroiden der Formel I

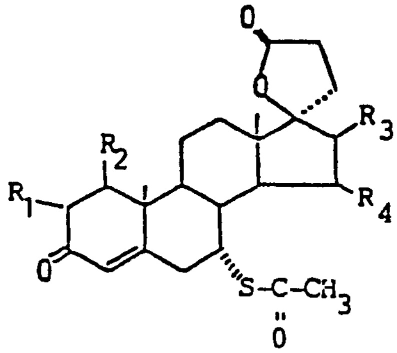

(I),

worin $R_1$, $R_2$, $R_3$ und $R_4$ Wasserstoff oder

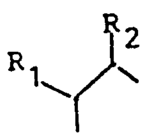 die Gruppe 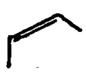 und

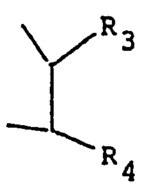 die Gruppe 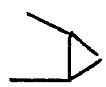 bedeuten,

indem man ein 3-Oxo-17α-pregna-4,6-dien-21,17--carbolacton der Formel II

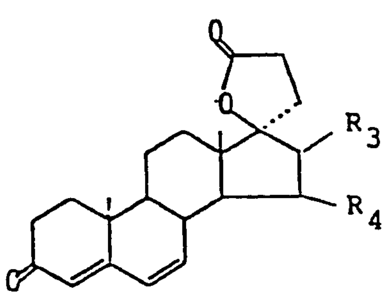

(II),

worin $R_3$ und $R_4$ die bereits genannten Bedeutungen haben, mit Thioessigsäure umsetzt, und das erhaltene Produkt gegebenenfalls dehydriert, dadurch gekennzeichnet, daß die Addition von Thioessigsäure in Gegenwart einer Lewissäure durchgeführt wird.

2. Verfahren gemäß Anspruch 1, dadurch gekennzeichnet, daß man als Lewissäure $BF_3$-Etherat verwendet.

**Claims**

1. Process for the preparation of 7α-acetylthiosteroids of formula I

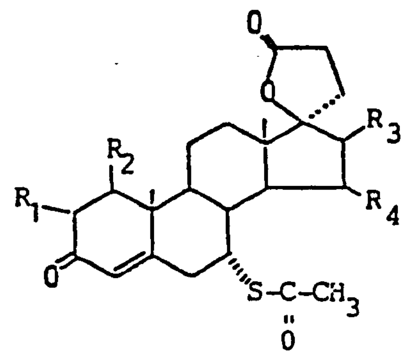

(I),

in which $R_1$, $R_2$, $R_3$ and $R_4$ represent hydrogen or

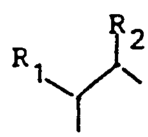 represents the group 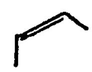 and

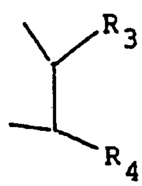 represents the group 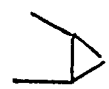

by reacting a 3-oxo-17α-pregna-4,6-diene-21,17--carbolactone of formula II

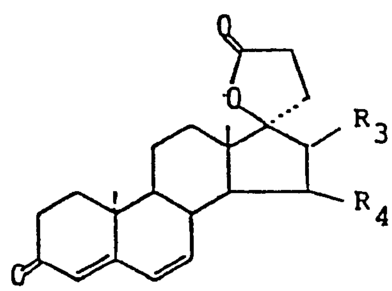

(II),

in which $R_3$ and $R_4$ have the meanings already mentioned, with thioacetic acid, and optionally dehydrogenating the product obtained, characterised in that the addition of thioacetic acid is carried out in the presence of a Lewis acid.

2. Process according to claim 1, characterised in that $BF_3$-etherate is used as the Lewis acid.

**Revendications**

1. Procédé pour préparer des acéthylthio-7α stéroïdes répondant à la formule I

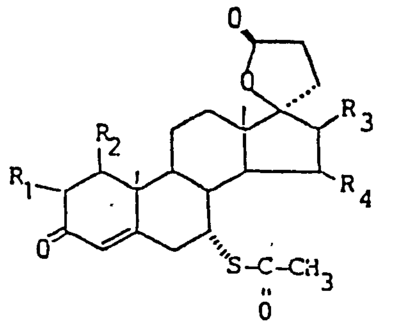

(I)

dans laquelle $R_1$, $R_2$, $R_3$ et $R_4$ représentent chacun l'hydrogène ou

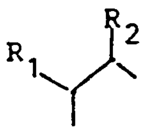 représente le radical 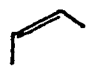 et

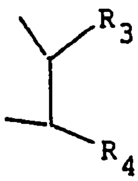 représente le radical 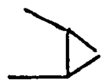

par réaction d'une oxo-3 17α-prégnadiène-4,6 carbolactone-21,17 répondant à la formule II

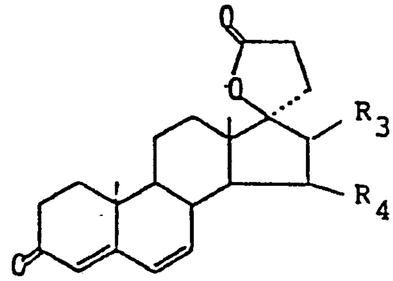

(II)

dans laquelle $R_3$ et $R_4$ ont les significations qui leur ont été données ci-dessus, avec l'acide thio-acétique, et déshydrogénation éventuelle du produit obtenu, procédé caractérisé en ce qu'on effectue l'addition de l'acide thio-acétique en présence d'un acide de Lewis.

2. Procédé selon la revendication 1, caractérisé en ce qu'on utilise, comme acide de Lewis, $BF_3$-oxyde de diéthyle.